# EUROPÄISCHE PATENTANMELDUNG

(11) **EP 1 913 930 A2**
(43) Veröffentlichungstag der Anmeldung: **23.04.2008**
(21) Anmeldenummer: 07020097.7
(22) Anmeldetag: 13.10.2007
(51) Int. Cl.: A61K 8/37, A61K 8/73, A61K 8/92, A61K 8/06, A61Q 19/00, A61Q 19/08

(54) **Hautfreundliche W/O-Emulsionen**

(30) Priorität: 18.10.2006 DE 102006049674
(71) Anmelder: Henkel Kommanditgesellschaft auf Aktien, 40589 Düsseldorf (DE)
(72) Erfinder: Hloucha, Matthias, 50677 Köln (DE); Frisoli, Christian, 41564 Kaarst (DE); Striepling, Gert-Lothar, 40591 Düsseldorf (DE); Struwe, Alexandra, 47877 Willich (DE)

(57) **Zusammenfassung**

Die vorliegende Erfindung betrifft Wasser-in-Öl-Emulsionen, enthaltend mindestens eine C₁₋₄-Alkylcellulose, mindestens einen Wachs, mindestens ein Öl sowie Wasser, und deren Verwendung in kosmetischen oder pharmazeutischen Zusammensetzungen, wobei die Wasser-in-Öl-Emulsion frei von herkömmlichen Emulgatoren ist.

## Beschreibung

Die vorliegende Erfindung betrifft Wasser-in-Öl-Emulsionen, enthaltend mindestens eine C₁₋₄-Alkylcellulose, mindestens einen Wachs, mindestens ein Öl sowie Wasser, und deren Verwendung in kosmetischen oder pharmazeutischen Zusammensetzungen, wobei die Wasser-in-Öl-Emulsion frei von herkömmlichen Emulgatoren ist.

Ein Problem der heute normalerweise verwendeten Wasser-in-Öl-Emulsionen (W/O-Emulsionen) für kosmetische oder pharmazeutische Anwendungen besteht darin, dass sie aufgrund der darin enthaltenen kurzkettigen Emulgatoren oder Tensiden zu Hautreizungen, Hautrötungen und/oder zu trockener Haut führen können.

Überraschenderweise wurde nun gefunden, dass W/O-Emulsionen, die mindestens eine C₁₋₄-Alkylcellulose, mindestens einen Wachs, mindestens ein Öl sowie Wasser enthalten, ganz besonders vorteilhafte Eigenschaften aufweisen. Hierbei kann überraschenderweise insbesondere auf die Verwendung kurzkettiger Coemulgatoren und darüber hinaus auf die Verwendung sonstiger Tenside und ebenso auf die Verwendung wasserlöslicher einwertiger Alkohole zur Stabilisierung der Emulsion verzichtet und damit das Problem der Hautreizung, Hautrötung und/oder trockener Haut vermieden werden.

Die Verwendung von C₁₋₄-Alkylcellulose in W/O-Emulsionen ist in der Literatur bereits beschrieben.

So werden in EP 1192937 W/O-Emulsionen beschrieben, die eine Ölphase, eine Wasserphase, Ethylcellulose sowie Emulgatoren enthalten. In US 4699779 werden wasserfeste Sonnencremes beschrieben, die neben Wasser, Ethylcellulose, einem UV-Absorber und Tensiden ein Alkalisierungs-Reagenz enthalten. In EP 0795318 werden W/O-Emulsionen beschrieben, die Ethylcellulose, Lösungsmittel, eine Ölphase, eine Wasserphase sowie wasserlösliche Polymere enthalten.

Überraschenderweise wurde festgestellt, dass die erfindungsgemäßen W/O-Emulsionen durch die Kombination aus C₁₋₄-Alkylcellulosen, Wachsen und Ölen schon mit sehr geringen Mengen an üblicherweise eingesetzten Tensiden und Emulgatoren, insbesondere ganz ohne diese, sowie schon mit sehr geringen Mengen an Alkoholen, insbesondere auch ganz ohne diese, stabilisiert werden können, das heißt, lagerstabil gegen Temperaturschwankungen, insbesondere bis zu + 50 °C und bis zu - 20 °C, gemacht werden können. Des Weiteren kann erfindungsgemäß vorteilhafter Weise auch auf den Einsatz anorganischer Pigmente, die zur Ausbildung so genannter Pickering-Emulsionen führen, verzichtet werden.

In einer bevorzugten Ausführungsform ist die erfindungsgemäße Zusammensetzung somit frei von normalerweise eingesetzten Tensiden und Emulgatoren, vor allem frei von solchen Tensiden und Emulgatoren, die ein Molekulargewicht kleiner 1000 g/mol besitzen, bevorzugt sogar frei von solchen, die ein Molekulargewicht kleiner 3000 g/mol besitzen, und besonders bevorzugt sogar frei von solchen, die ein Molekulargewicht kleiner 5000 g/mol besitzen, bzw. sind diese jeweils nur in sehr geringen Mengen, insbesondere zu weniger als 0,2, vorzugsweise zu weniger als 0,1, besonders bevorzugt zu weniger als 0,05, insbesondere zu weniger als 0,03, vor allem zu weniger als 0,01 Gew.-%, enthalten. Die Zusammensetzung kann hierbei unabhängig voneinander frei sein von kationischen, anionischen, amphoteren oder nichtionischen Tensiden, ist jedoch besonders bevorzugt frei von all diesen, insbesondere jedoch frei von kationischen und anionischen Tensiden, hierbei vor allem frei von kurzkettigen Emulgatoren und Tensiden bzw. sind diese in nur sehr geringen Mengen, wie zuvor angegeben, enthalten. Unter kurzkettigen Emulgatoren und Tensiden sind erfindungsgemäß entsprechend Emulgatoren und Tenside mit einem Molgewicht kleiner 1000 g/mol, bevorzugt solche mit einem Molgewicht kleiner 3000 g/mol, besonders bevorzugt solche mit einem Molgewicht kleiner 5000 g/mol zu verstehen.

Die erfindungsgemäße Zusammensetzung ist des weiteren vorzugsweise frei von Pigmenten, insbesondere anorganischen Pigmenten, mit einer Partikel-Größe von kleiner als 500 nm, vorzugsweise frei von Pigmenten mit einer Partikel-Größe von kleiner als 10 Mikrometer, und besonders bevorzugt sogar frei von Pigmenten mit einer Partikel-Größe von kleiner als 100 Mikrometer. Die erfindungsgemäße Zusammensetzung ist des Weiteren vorzugsweise frei von Alkalisierungs-Reagenzien und frei von aromatischen Harzen.

Ein erster Gegenstand der vorliegenden Erfindung sind daher Wasser-in-Öl-Emulsionen (W/O-Emulsionen), enthaltend mindestens eine C₁₋₄-Alkylcellulose, mindestens einen Wachs, mindestens ein Öl sowie Wasser, dadurch gekennzeichnet, dass die W/O-Emulsionen Tenside und Emulgatoren mit einem Molekulargewicht kleiner 1000 g/mol, bevorzugt kleiner 3000 g/mol, besonders bevorzugt kleiner 5000 g/mol, in einer Menge von weniger als 0,2, vorzugsweise von weniger als 0,1, besonders bevorzugt von weniger als 0,05, insbesondere von weniger als 0,03, vor allem von weniger als 0,01 Gew.-%, enthalten.

Gegenstand der vorliegenden Erfindung sind daher des Weiteren kosmetische oder pharmazeutische Zusammensetzungen, die aus diesen W/O-Emulsionen bestehen oder diese enthalten.

Unter Tensiden und Emulgatoren mit einem Molekulargewicht kleiner 1000 g/mol, bevorzugt kleiner 3000 g/mol, besonders bevorzugt kleiner 5000 g/mol, die erfindungsgemäß weniger bevorzugt sind und die in erfindungsgemäß bevorzugten Zusammensetzungen nur in sehr geringen Mengen, insbesondere zu weniger als 0,2, vorzugsweise zu weniger als 0,1, besonders bevorzugt zu weniger als 0,05, insbesondere zu weniger als 0,03, vor allem zu weniger als 0,01 Gew.-%, enthalten sind oder aber von denen erfindungsgemäß besonders bevorzugte Zusammensetzungen frei sind, sind erfindungsgemäß insbesondere solche zu verstehen, die der folgenden Definition genügen:

Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside, bestehend aus einem hydrophoben Rest und einer negativ geladenen hydrophilen Kopfgruppe, amphotere Tenside, die sowohl eine negative als auch eine kompensierende positive Ladung tragen, kationische Tenside, die neben einem hydrophoben Rest eine positiv geladene hydrophile Gruppe aufweisen, und nichtionische Tenside, die keine Ladungen, sondern starke Dipolmomente aufweisen und in wässriger Lösung stark hydratisiert sind. Weitergehende Definitionen und Eigenschaften von Tensiden finden sich in "H.-D.Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift.

Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D. Dörfler, Grenzflächen- und Kolloidchemie, VCH Verlagsgesellschaft mbH. Weinheim, 1994".

Anionische Tenside und Emulgatoren sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für erfindungsgemäß weniger bevorzugte anionische Tenside und Emulgatoren sind, jeweils in Form der Natrium-, Kalium-, Calcium-, Magnesium-, Zink- und Ammonium- sowie der Mono-, Di-und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-1), 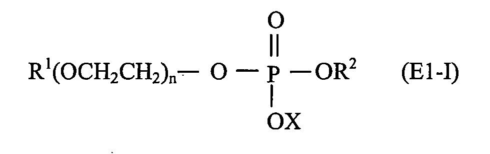
- in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄- Kohlenwasserstoffrest, steht,
- sulfatierte Fettsäurealkylenglykolester der Formel R⁷CO(AlkO)ₙSO₃M, in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (E1-III) 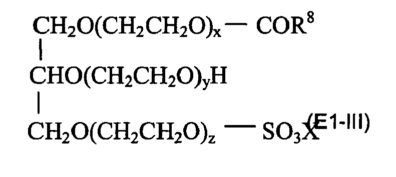
- in der R⁸CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht,
- Amidethercarbonsäuren wie sie in der EP 0 690 044 beschrieben sind,
- Kondensationsprodukte aus C₈-C₃₀- Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder - SO₃⁽⁻⁾ -Gruppe tragen. Besonders häufig eingesetzte zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein besonders häufig eingesetztes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈- C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für besonders häufig eingesetzte ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol-Typen (Cognis),
- alkoxylierte Triglyceride,
- alkoxylierte Fettsäurealkylester der Formel R¹CO-(OCH₂CHR²)_{w}OR³, in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungsprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß der Formel R⁴O-[G]ₚ, in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht,
- Zuckertenside vom Typ der Fettsäure-N-alkylpolyhydroxyalkylamide, ein nichtionisches Tensid der Formel R⁵CO-NR⁶-[Z], in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.
Häufig eingesetzte nichtionische Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),

Häufig eingesetzte kationische Tenside sind solche vom Typ der quaternären Ammoniumverbindungen, der Esterquats und der Amidoamine. Besonders häufig eingesetzte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quatemium-83 bekannten Imidazolium-Verbindungen.

Bei den häufig eingesetzten Esterquats handelt es sich um bekannte Stoffe, die sowohl mindestens eine Esterfunktion als auch mindestens eine quartäre Ammoniumgruppe als Strukturelement enthalten, insbesondere quaternierte Estersalze von Fettsäuren mit Triethanolamin, quaternierte Estersalze von Fettsäuren mit Diethanolalkylaminen und quatemierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart® F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine besonders häufig eingesetzte Verbindung aus dieser Substanzgruppe stellt das unter der Bezeichnung Tegoamid^{®} S 18 im Handel erhältliche Stearamidopropyl-dimethylamin dar.

Geringe Mengen an Tensiden und Emulgatoren werden häufig durch Parfumöl- oder Wirkstoffzubereitungen eingetragen, bei denen das Parfumöl oder der Wirkstoff schlecht wasserlöslich ist und mit Hilfe eines geringen Zusatzes an Tensiden oder Emulgatoren solubilisiert oder voremulgiert wird, um seine homogene Einarbeitung in die erfindungsgemäße Zusammensetzung zu gewährleisten. Dementsprechend kann es erfindungsgemäß bevorzugt sein, dass bis zu 0,2, bis zu 0,1, bis zu 0,05, bis zu 0,03 oder bis zu 0,01 Gew.-%, jeweils bezogen auf die gesamte erfindungsgemäße Zusammensetzung, an Tensiden oder Emulgatoren, die aus Parfum- oder Wirkstoffzubereitungen stammen, enthalten sind.

Der vorstehend diskutierte bevorzugte niedrige Gehalt an Tensiden und Emulgatoren gilt in einer weiteren bevorzugten Ausführungsform nicht für solche oberflächenaktiven Substanzen, die in die erfindungsgemäßen Zusammensetzungen als Bestandteile von Liposomen, uni- , pauci- und/oder multilamellaren Vesikeln eingebracht und/oder enthalten sind. Es wurde nämlich überraschenderweise als weiterer Vorteil der erfindungsgemäßen Zusammensetzungen festgestellt, dass sie besonders gut zur Stabilisierung von Liposomen geeignet sind und dadurch den Transport von Liposomen, die Wirkstoffe enthalten können, in die Haut ermöglichen, ohne dass die Liposomen bereits in der Zusammensetzung in nennenswerter Anzahl geschädigt werden, wie dies bei Verwendung klassischer Tenside oftmals geschieht, wenn diese sich in die Liposomen- oder Vesikellamellen einlagern und diese dadurch destabilisieren. Weitere bevorzugte erfindungsgemäße Zusammensetzungen sind daher dadurch gekennzeichnet, dass Liposomen, uni-, pauci- und/oder multilamellaren Vesikeln enthalten sind.

Bei den Liposomen kann es sich hierbei um jede beliebige Art von Liposomen handeln. Besonders bevorzugt sind Liposomen und/oder Vesikeln auf der Basis von Phospholipiden, insbesondere auf der Basis von Lecithinen bzw. Phosphatidylcholinen. Die Liposomen oder Vesikeln enthalten vorzugsweise hydrophile Wirkstoffe.

Gegenstand der vorliegenden Anmeldung ist daher weiterhin die Verwendung erfindungsgemäßer Zusammensetzungen, die Liposomen, uni- , pauci- und/oder multilamellaren Vesikeln enthalten, zur Speicherung von Wirkstoffen sowie zum Transport von Wirkstoffen in die Haut.

Weitere besondere Vorteile der erfindungsgemäßen Zusammensetzungen sind, dass sie hinsichtlich der Probleme Hautreizung (Hautirritation), Hautrötung oder trockene Haut ein besseres Verhalten zeigen als die herkömmlicher Weise verwendeten Zubereitungen und dass vorzugsweise diese Probleme vollständig vermieden werden können. Die erfindungsgemäßen Zusammensetzungen sind daher besonders gut geeignet zur Verwendung bei sensitiver, empfindlicher oder trockener Haut bzw. in Bereichen, in denen es leicht zu Irritationen kommen kann, beispielsweise im Bereich der Augen. Sie sind daher ebenfalls geeignet zur Vermeidung trockener Haut und zur Vermeidung von Hautirritationen.

Weitere Vorteile der erfindungsgemäßen Zusammensetzungen sind hohe Stabilität der Zusammensetzungen gegen Phasenseparation bei verschiedenen Lagerbedingungen, ein gutes Hautgefühl, was insbesondere auf die gute Verteilbarkeit, das gute Gleitverhalten, auf fehlende Klebrigkeit, das Ausbleiben von Rückständen und das gute Einziehverhalten zurückzuführen ist, sowie gute Anwendungseigenschaften wie das Fehlen des Weißeleffekts (beim Verreiben tritt keine vorübergehende Weißfärbung auf, die auf Schaumbildung zurückzuführen ist).

Aufgrund ihrer vorteilhaften Eigenschaften sind die erfindungsgemäßen Zusammensetzungen auch zur Behandlung extrinsisch und/oder intrinsisch gealterter Haut gut geeignet.

In einer erfindungsgemäß bevorzugten Ausführungsform beträgt der Anteil an Ethylcellulose 0,1 - 5,0, bevorzugt 0,5-2,0 Gew.-%, der Anteil an Wachsen 0,1-5,0, bevorzugt 1,0-4,0 Gew.-% und der Anteil an Ölen 1 - 40, bevorzugt 2 - 35, besonders bevorzugt 5 - 30 Gew.-%.

Der pH-Wert der erfindungsgemäßen Zusammensetzungen beträgt vorzugsweise von 5,0 bis 7,0, besonders bevorzugt von 5,3 bis 6,5.

Bei der erfindungsgemäßen kosmetischen oder pharmazeutischen Zusammensetzung kann es sich insbesondere um eine Lotion, eine Creme oder ein Salbe handeln.

In einer bevorzugten Ausführungsform handelt es sich um eine Zusammensetzung zur Anwendung in empfindlichen, leicht irritierbaren Bereichen, insbesondere im Bereich der Augen oder im Intimbereich oder in anderen Bereichen der Schleimhaut. Es kann sich entsprechend beispielsweise um eine Augensalbe, Augencreme oder um eine Intimsalbe handeln.

Als Applikationsort kommt entsprechend die Haut jedes Körperbereichs in Frage, insbesondere jedoch die Gesichtshaut, besonders im Bereich der Augen, oder die Schleimhaut, insbesondere im Intimbereich.

### C₁₋₄-Alkylcellulose

Bei der C₁₋₄-Alkylcellulose handelt es sich um eine Cellulose, die mit Methanol, Ethanol, n-Propanol, i-Propanol, n-Butanol, i-Butanol oder tert.-Butanol oder Mischungen davon verethert ist. Besonders bevorzugt ist erfindungsgemäß die Ethylcellulose.

Die C₁₋₄-Alkylcellulose ist in den erfindungsgemäßen Zusammensetzungen vorzugsweise in einer Menge 0,1 - 5,0, bevorzugt 0,5 - 2,0 Gew.-%, enthalten.

### Wachse

Bei dem erfindungsgemäß einzusetzenden Wachs kann es sich insbesondere um einen natürlichen Wachs, einen chemisch modifizierten Wachs oder einen synthetischen Wachs oder Mischungen davon handeln. Bei dem natürlichen Wachs kann es sich insbesondere um einen pflanzlichen Wachs, einen tierischen Wachs oder um einen Mineralwachs oder um Mischungen davon handeln. Bei dem chemisch modifizierten Wachs kann es sich insbesondere um einen Hartwachs handeln.

Als Wachse können erfindungsgemäß insbesondere eingesetzt werden feste Paraffine oder Isoparaffine, Pflanzenwachse wie Candelillawachs, Carnaubawachs, Espartograswachs, Guarumawachs, Japanwachs, Korkwachs, Reiskeimölwachs, Lorbeerbaumwachs, Kapokwachs, Baumwollwachs, Zuckerrohrwachs, Ouricurywachs, Montanwachs, Sonnenblumenwachs sowie Fruchtwachse, tierische Wachse wie beispielsweise Bienenwachse und andere Insektenwachse, Walrat, Schellackwachs, Wollwachs (Lanolin) und Bürzelfett, weiterhin Mineralwachse, wie z. B. Ceresin und Ozokerit oder die petrochemischen Wachse, wie z. B. Petrolatum, Paraffinwachse, Isoparaffinwachse, Mikrowachse aus Polyethylen oder Polypropylen und Polyethylenglycolwachse (POE-Wachse), insbesondere POE-Lanolinalkoholether, POE-Lanolinalkoholacetat, POE-Cholesterinether, Lanolinfettsäurepolyethylenglykol oder hydrierter POE-Lanolinalkoholether. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Triglyceride gesättigter und gegebenenfalls hydroxylierter C₁₆₋₃₀- Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs^{®}) oder Polyolen mit 2 - 6 C-Atomen, Fettsäuremonoalkanolamide mit einem C₁₂₋₂₂-Acylrest und einem C₂₋₄-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen.

Der Wachs kann insbesondere ausgewählt sein aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere kann der Wachs hierbei aus der Gruppe der C₁₆₋₃₆-Alkylstearate, der C₁₀₋₄₀-Alkylstearate, der C₂₋₄₀-Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Bevorzugte Wachskomponenten sind die Ester aus gesättigten, einwertigen C₂₀-C₆₀-Alkoholen und gesättigten C₈-C₃₀-Monocarbonsäuren, insbesondere ein C₂₀-C₄₀-Alkylstearat, das unter dem Namen Kesterwachs^{®} K82H von der Firma Koster Keunen Inc. erhältlich ist.

In einer erfindungsgemäß besonders bevorzugten Ausführungsform wird als Wachs Ozokerit, Cetylpalmitat, Wollwachs, Bienenwachs oder Paraffinwachs eingesetzt.

Der Wachs oder die Wachskomponenten sollten bei einem Druck von einem Bar und einer Temperatur von 25° C fest sein, jedoch im Bereich von 35 - 95°C schmelzen, wobei ein Schmelzbereich zwischen 45 und 85 °C bevorzugt ist.

Natürliche, chemisch modifizierte und synthetische Wachse können alleine oder in Kombination eingesetzt werden.

Der Wachs ist in den erfindungsgemäßen Zusammensetzungen vorzugsweise in einer Menge von 0,1 - 5,0, bevorzugt 1,0 -4,0 Gew.-% enthalten.

### Öle

Die kosmetischen oder pharmazeutischen Zusammensetzungen enthalten neben den Wachsen als weitere Fettstoffe Öle. Als Öle sind erfindungsgemäß insbesondere solche Fettstoffe zu verstehen, die bei Raumtemperatur, d.h. bei einem Druck von einem Bar und einer Temperatur von 25°C, flüssig sind. Die Öle sind insbesondere ausgewählt aus der Gruppe bestehend aus verzweigten Fettalkoholen sowie natürlichen und synthetischen kosmetischen Ölkomponenten.

Bei dem Öl kann es sich insbesondere um ein unpolares oder polares flüssiges Öl handeln, das natürlich oder synthetisch sein kann. Die Ölkomponente kann insbesondere ausgewählt sein aus
- pflanzlichen Ölen, insbesondere Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssigen Paraffinölen, Isoparaffinölen, z. B. Isohexadecan und Isoeicosan, aus hydrogenierten Polyalkenen, insbesondere Poly-1-decenen (im Handel erhätlich als Nexbase 2004, 2006 oder 2008 FG (Fortum, Belgien)), aus synthetischen Kohlenwasserstoffen, z.B. 1,3-Di-(2-ethyl-hexyl)-cyclohexan (Cetiol® S), sowie aus flüchtigen und nichtflüchtigen Siliconölen, die cyclisch, wie z. B. Decamethylcyclopentasiloxan und Dodecamethylcyclohexasiloxan, oder linear sein können, z. B. lineares Dimethylpolysiloxan, im Handel erhältlich z. B. unter der Bezeichnung Dow Corning^{®} 190, 200, 244, 245, 344, 345 oder 350 und Baysilon^{®} 350 M,
- Di-n-alkylethern mit insgesamt zwischen 12 bis 36 C-Atomen, insbesondere 12 bis 24 C-Atomen, wie beispielsweise Di-n-octylether (im Handel erhältlich als Cetiol® OE), Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether.
- Esterölen. Unter Esterölen sind zu verstehen die Ester von C₆-C₃₀-, insbesondere C₆-C₂₂-Fettsäuren, mit C₂-C₃₀-, insbesondere C₂-C₂₄-Fettalkoholen. Beispiele für eingesetzte Fettsäurenanteile in den Estern sind Capronsäure, Caprylsäure, 2-Ethyl-hexansäure, Caprinsäure, Laurinsäure, Isotridecansäure, My-ri-stinsäure, Palmitinsäure, Palmitoleinsäure, Stearinsäure, Isostearinsäure, Ölsäure, Elaidinsäure, Petroselinsäure, Linolsäure, Linolensäure, Elaeo-stearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosyn-these oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassi-dylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Erfindungsgemäß einsetzbar sind insbesondere Isopropylmyristat (Rilanit® IPM), Isononansäure-C16-18-alkylester (Cetiol® SN), 2-Ethylhexylpalmitat (Cegesoft® 24), Stearinsäure-2- ethylhexylester (Cetiol® 868), Cetyloleat (Cetiol®V), Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V),
- Dicarbonsäureestern wie Di-n-butyladipat, Di-(2-ethylhexyl)-adipat, Di-(2-ethylhexyl)-succinat und Diisotridecylacelaat sowie Diolester wie Ethylenglykol-dioleat, Ethylenglykoldiisotridecanoat, Propylenglykol-di(2-ethylhexanoat), Propylenglykoldiisostearat, Propylenglykoldipelargonat, Butandioldiisostearat, Neopentylglykoldicaprylat,
- symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol® CC),
- Mono-, Di- und Trifettsäureestern, insbesondere Trifettsäureestern, von gesättigten und/oder ungesättigten linearen und/oder verzweigten Fettsäuren, insbesondere C₆-C₂₂-Fettsäuren, mit Glycerin, wie beispielsweise Triglyceride der Caprinsäure und/oder Caprylsäure, Monomuls® 90-018, Monomuls® 90-L12 oder Cutina® MD.

Die polare Ölkomponente kann weiterhin ausgewählt sein aus verzweigten Fettalkoholen, d.h. aus verzweigten Alkanolen, insbesondere aus Alkanolen mit 8-18 C-Atomen, die durch Alkylreste mit 4-12 C-Atomen, ein- oder mehrfach, vorzugsweise einfach, insbesondere am Kohlenstoffatom 2 substituiert sind, wie z. B. Guerbet-Alkohole mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethem oder Di-n-alkylethem mit jeweils insgesamt 12 - 24 C-Atomen, insbesondere Di-n-octylether (Cetiol^{®}OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol^{®} APM), PPG-14-Butylether (Ucon Fluid^{®} AP), PPG-15-Stearylether (Arlamol^{®} E), PPG-9-Butylether (Breox^{®} B25) und PPG-10-Butandiol (Macol^{®} 57).

Erfindungsgemäß ist jede beliebige Kombination der oben genannten Öle einsetzbar.

In einer erfindungsgemäß bevorzugten Ausführungsform umfasst das Öl mindestens eines der oben genannten polaren Öle. Als besonders vorteilhaft hat sich erfindungsgemäß der Einsatz von verzweigten Alkanolen herausgestellt, insbesondere von Alkanolen umfassend einen linearen C₈₋₁₈-Alkanol, der durch C₄₋₁₂-Alkylreste ein- oder mehrfach, vorzugsweise einfach, insbesondere am Kohlenstoffatom 2 substituiert ist, wie z. B. Guerbet-Alkohole mit einer einzigen Verzweigung am Kohlenstoffatom 2 wie 2-Hexyldecanol, 2-Octyldodecanol, Isotridecanol und Isohexadecanol. Diese verzweigten Alkanole werden erfindungsgemäß auch "verzweigte Fettalkohole" genannt.

In einer weiteren erfindungsgemäß bevorzugten Ausführungsform wird eine Kombination des zuvor genannten verzweigten Alkanols mit mindestens einem weiteren Öl eingesetzt, denn es hat sich erfindungsgemäß des Weiteren herausgestellt, dass es bei Verzicht auf Emulgatoren von weiterem Vorteil ist, eine Mischung aus einem verzweigten Alkanol und mindestens einem weiteren Öl einzusetzen.

Das Öl, und insbesondere der verzweigte Fettalkohol, ist in den erfindungsgemäßen Zusammensetzungen vorzugsweise in einer Menge von 1-40 Gew.-%, besonders bevorzugt in einer Menge von 2-35 Gew.-%, vor allem in einer Menge von 5-30 Gew.-% enthalten.

Die erfindungsgemäße kosmetische oder pharmazeutische Zusammensetzung kann auch weitere Bestandteile als die zuvor genannten enthalten. In einer bevorzugten Ausführungsform enthält sie mindestens eine der im folgenden aufgezählten Substanzen. Sie kann auch jede beliebige Kombination der im folgenden aufgezählten Bestandteile enthalten.

### Wasserlösliche Polymere

Als wasserlösliche Polymere eignen sich alle in Wasser bei 20° C in Mengen von wenigstens 0,5 Gew.-% löslichen natürlichen oder synthetischen hochmolekularen Stoffe mit wiederkehrenden Strukturelementen. Als natürliche Polymere werden dabei wasserlösliche Polysaccharide oder die wasserlöslichen Derivate von wasserunlöslichen Polysacchariden verstanden. Geeignete natürliche Polymere sind z.B. Stärke, Guar, Celluloseether, z.B. Methylcellulosen, Hydroxyethylcellulosen oder Carboxymethylcellulosen.

Geeignete synthetische Polymerverbindungen sind z.B. Polyvinylalkohole, Polyvinylpyrrolidon, Polyacrylamide, Polyvinylacetate und Polyalkylenglykole mit Molekulargewichten von mehr als 1000 D. Auch wasserlösliche Copolymerisate der Acrylsäure, des Acrylamids, des Vinylpyrrolidons und anderer wasserlöslicher Monomeren mit nicht-wasserlöslichen Comonomeren sind als Polymerverbindungen im Sinne der vorliegenden Erfindung geeignet.

Bevorzugt sind die wasserlöslichen Polymere ausgewählt aus Polyethylenglykolen, Polypropylenglykolen, EO-PO- Polyalkylenglykolen und Polyvinylpyrrolidon mit einem mittleren Molekulargewicht von mindestens 1000 D und besonders bevorzugt von 5000 bis 50000 D, wobei sich die genannten Werte auf mittels der Gelpermeation bestimmte Molekulargewichtswerte beziehen.

Als wasserlösliche Polymere besonders bevorzugt sind Polyethylenglykol und Polyvinylpyrrolidon mit einem mittleren Molekulargewicht im Bereich von 5000 bis 50000 D.

Wasserlösliche Polymere, und insbesondere Polyethylenglykol oder Polyvinylpyrrolidon, werden vorzugsweise in einer Menge von bis zu 5 Gew.-%, besonders bevorzugt in einer Menge von 0,1 - 4,0 Gew.-%, insbesondere in einer Menge von 0,2 -1,0 Gew.-%, eingesetzt.

### Salz

Erfindungsgemäß einsetzbare anorganische Salze sind vorzugsweise ausgewählt aus der Gruppe umfassend farblose wasserlösliche Halogenide, Sulfate, Carbonate, Hydrogencarbonate und/oder Phosphate der Alkalimetalle, der Erdalkalimetalle und/oder des Aluminiums.

Bei den erfindungsgemäß einsetzbaren organischen Salzen handelt es sich insbesondere um farblose wasserlösliche Alkalimetall-, Erdalkalimetall-, Ammonium-, Aluminium- und/oder Über-gangsmetallsalze der Carbonsäuren. Vorzugsweise sind die Salze ausgewählt aus der Gruppe umfassend Formiat, Acetat, Propionat, Citrat, Malat, Tartrat, Succinat, Malonat, Oxalat, Lactat sowie Gemische derselben.

Besonders bevorzugt wird ein anorganisches Salz eingesetzt.

Salze, und insbesondere anorganische Salze, werden vorzugsweise in einer Menge von bis zu 5 Gew.-%, besonders bevorzugt in einer Menge von 0,1-5,0 Gew.-%, insbesondere in einer Menge von 0,5-2,0 Gew.-%, eingesetzt.

### Hydrophob modifizierte Polysaccharide

Bei dem Grundgerüst des hydrophob modifizierten Polysaccharids handelt es sich erfindungsgemäß vorzugsweise um ein wasserlösliches Polysaccharid, das auch teilweise mit gegebenenfalls substituiertem Methanol und/oder Ethanol verethert sein kann. Das wasserlösliche Polysaccharid kann jede beliebige Struktur besitzen. So kann es beispielsweise linear, verzweigt, kammartig und/oder sternförmig sein, wobei jedoch lineare Polysaccharide bevorzugt sind.
Bei dem Grundgerüst des hydrophob modifizierten Polysaccharids kann es sich ferner auch um ein Copolymer oder um ein Block-Copolymer unterschiedlicher Monosaccharid-Einheiten und/oder auf unterschiedliche Weise miteinander verknüpfter Monosaccharid-Einheiten handeln.

Erfindungsgemäß verwendbare Polysaccharid-Grundgerüste sind beispielsweise ausgewählt aus der Gruppe bestehend aus Polyglucose, insbesondere Cellulose, Amylose, Amylopektin oder Dextrine, und Polyfructose sowie Methyl-, Ethyl-, Hydroxymethyl- oder Hydroxyethyl-modifizierten Derivaten dieser Verbindungen. Erfindungsgemäß besonders bevorzugte Polysaccharid-Grundgerüste sind Methylcellulose, Hydroxyethylcellulose und lineare Polyfructose, insbesondere Inulin, ganz besonders bevorzugt ist Methylcellulose.

Das Polysaccharid umfasst in einer bevorzugten Ausführungsform im Mittel von 5 bis 1000, besonders bevorzugt von 10 bis 500, vor allem von 20 bis 100, Monosaccharid-Einheiten.

Die hydrophobe Modifizierung wird vorzugsweise erreicht durch C₃₋₂₂-Alkylgruppen, insbesondere durch C₃₋₁₈-Alkylgruppen, besonders bevorzugt durch C₃₋₁₆-Alkylgruppen. Die Alkylgruppen können linear oder verzweigt, gesättigt oder ungesättigt sein, sind jedoch vorzugsweise linear und ungesättigt.
Die hydrophoben Gruppen sind vorzugsweise über eine Alkyl-Etherbindung oder über eine Alkyl-Urethanbindung vorzugsweise an die Hydroxygruppen des Polysaccharids gebunden. Die hydrophoben Gruppen können aber auch über einen Linker an das Polysaccharid-Grundgerüst gebunden sein, beispielsweise über einen Polyether-Linker, insbesondere einen Polyethylenglykol-Linker.

Das hydrophob modifizierte Polysaccharid besitzt in einer bevorzugten Ausführungsform ein Molekulargewicht von 5000 bis 500.000 g/mol, besonders bevorzugt von 5000 bis 100.000 g/mol.

Der Modifizierungsgrad des hydrophob modifizierten Polysaccharids, d.h. der Quotient aus Anzahl an hydrophob modifizierenden Gruppen und modifizierbaren Gruppen, beträgt vorzugsweise von 0,01 bis 0,9, besonders bevorzugt von 0,03 bis 0,15.

Die hydrophoben Gruppen sowie unabhängig davon das Polysaccharid-Rückgrat können gegebenenfalls auch ein- oder mehrfach substituiert sein, insbesondere durch Reste ausgewählt aus Halogen, insbesondere Fluor, Chlor oder Brom, Hydroxy, Alkoxy, insbesondere C₁₋₆-Alkoxy, Amino, Alkylamino, insbesondere C₁₋₆-Alkylamino, Aryl, insbesondere C₆₋₁₀-Aryl, Arylalkyl, insbesondere C₆₋₁₀-Aryl-C₁₋₆-alkyl, Carboxy, Carboxyester, insbesondere Carboxy-C₁₋₆-alkylester, und cycloaliphatischen Resten, wobei die Hydroxy-Gruppe als Substituent bevorzugt ist. Bevorzugt handelt es sich bei den hydrophob modifizierten Polysacchariden um nichtionische Verbindungen.

Erfindungsgemäß eingesetzte hydrophob modifizierte Polysaccharide sind grenzflächenaktiv und senken die Grenzflächenspannung von Wasser gegenüber Luft und von Wasser gegenüber Öl deutlich ab. Bevorzugt ist hierbei eine Absenkung der Oberflächenspannung einer 1%igen Lösung auf kleiner als 60 mN/m und die Absenkung der Grenzflächenspannung einer 1%igen Lösung gegenüber kosmetischen organischen Ölen, insbesondere gegenüber Decyl Oleate, auf Werte unter 25, vorzugsweise unter 20 mN/m (gemessen bei 25°C nach folgenden Methoden: Ring-Tensio-Meter (Fa. Lauda) und Drop-Shape-Analysis (Fa. Krüss)).

Erfindungsgemäß bevorzugt eingesetzte hydrophob modifizierte Polysaccharide sind offenbart in EP 964054 A1 sowie in Biomacromolecules (2001, Band 2, S. 1256-1259).

Erfindungsgemäß besonders bevorzugt eingesetzte hydrophob modifizierte Polysaccharide sind Polysaccharid-N-alkylurethane der Firma Orafti, vor allem Inulin Lauryl Carbamate (Inutec SP-1, Fa. Orafti), sowie hydrophob modifizierte Hydroxyethylcellulose der Firma Hercules, insbesondere Cetyl Hydroxyethylcellulose (Natrosol Plus CS 330, Polysurf, Fa. Hercules).

Erfindungsgemäß besonders bevorzugte hydrophob modifizierte Polysaccharide sind des weiteren Hydroxy-C₃₋₆-alkyl-modifizierter Cellulose, insbesondere Hydroxypropylcellulose und Hydroxypropylmethylcellulose, wobei Hydroxypropylmethylcellulose erfindungsgemäß ganz besonders bevorzugt ist. Die Hydroxyprpopylmethylcellulose ist im Handel etwa unter dem Namen Pharmacoat 606 (Shin-Etsu) erhältlich.

Die Menge an hydrophob modifizierten Polysacchariden in den erfindungsgemäßen Zusammensetzungen beträgt vorzugsweise von 0,1 bis 5,0, besonders bevorzugt von 0,1 bis 3,0, vor allem von 0,5 und 2,0 Gew.-%.

### Hydrophob modifizierte Polymere

Die erfindungsgemäße Zusammensetzung kann neben hydrophob modifizierten Polysacchariden auch weitere hydrophob modifizierte Polymere enthalten. Diese weiteren hydrophob modifizierten Polymeren können beispielsweise ausgewählt sein aus hydrophob modifizierten Polysäuren, hydrophob modifizierten Polyamiden, hydrophob modifizierten Polyalkoholen und Mischungen davon.

Bevorzugt handelt es sich bei diesen hydrophob modifizierten Polymeren um hydrophob modifizierte Polyacrylsäure- und Polymethacrylsäureamide oder hydrophob modifizierte Polyacrylate und/oder Polymethacrylate, wobei hydrophob modifizierte Polyacrylate und/oder Polymethacrylate besonders bevorzugt sind. Unter Polyacrylaten und Polymethacrylaten werden erfindungsgemäß sowohl die Salze als auch die Ester von Polyacrylsäuren und Polymethacrylsäuren verstanden. Bevorzugte Salze der Polyacrylsäuren und Polymethacrylsäuren sind die Natrium- und die Ammoniumsalze. Bevorzugte Ester der Polyacrylsäuren und Polymethacrylsäuren sind die Methyl-, Ethyl-, Hydroxyethyl-, Propyl- und Hydroxypropylester.

Die hydrophob modifizierten Polymere haben vorzugsweise die Eigenschaft, dass ihre 0,5%igen wässrigen Lösungen eine Fließgrenze von größer gleich 0,1 Pa, besonders bevorzugt größer gleich 0,5 Pa erreichen und deren 2%ige wässrige Lösungen eine Fließgrenze von größer gleich 10 Pa, besonders bevorzugt größer gleich 100 Pa aufweisen.

Als Beispiele für erfindungsgemäß verwendbare hydrophob modifizierte Polymere seien genannt von Rohm und Haas Acusol 801 S, Acusol 820, Aculyn 22 (Acrylates/Steareth-20 Methacrylate Copolymer) und Aculyn 28 (Acrylates/Beheneth-25 Methacrylate Copolymer), von Noveon Pemulen TR-1, Pemulen TR-2, Carbopol ETD2020 und Carbopol Ultrez 20 Polymer (jeweils Acrylates/C10-30 Alkyl Acrylate Crosspolymer) und Carbopol Aqua SF-1, von National Starch Structure 3001 (Acrylates/Ceteth-20 Itaconate Copolymer) und Structure 2001 (Acrylates/Palmeth-25 Itaconate Copolymer), von 3V Sigma Synthalen W2000 (Acrylates/Palmeth-25 Acrylate Copolymer) und von Clariant Aristoflex HMB (Ammonium Acryloyldimethyltaurate/Beheneth-25 Methacrylate Crosspolymer).

Die Gesamtmenge an solchen hydrophob modifizierten Polymeren in den erfindungsgemäßen Zusammensetzungen beträgt, soweit vorhanden, vorzugsweise bis zu 2,0 Gew.-%, insbesondere von 0,1 -1,5 Gew.-%, besonders bevorzugt von 0,2 - 1,0 Gew.-%.

### Hydrophil modifizierte Silikone

Erfindungsgemäße Zusammensetzungen können ferner gegebenenfalls auch hydrophil modifizierte Silikone enthalten. Auch wenn diese Verbindungen Emulgator-Eigenschaften aufweisen, sind diese Verbindungen nicht als Emulgatoren im Sinne der vorliegenden Anmeldung anzusehen, da sie keine irritativen Eigenschaften aufweisen.

Hydrophil modifizierte Silikone trugen bis vor kurzem die relativ undifferenzierte INCI-Bezeichnung Dimethicone Copolyol bzw., wenn sie von Methylgruppen verschiedene Alkylsubstituenten enthielten, die Bezeichnung Alkyl Dimethicone Copolyol, z. B. Cetyl Dimethicone Copolyol. Mittlerweile beinhaltet die INCI-Bezeichnung Angaben über die Zusammensetzung der hydrophilen Substituenten, insbesondere über die Anzahl an Polyethylenglycol- und/oder Polypropylenglycol-Einheiten, z.B. PEG-3 Dimethicone oder PEG/PPG-25/25 Dimethicone.

Im Sinne der vorliegenden Erfindung steht die Bezeichnung ,,Dimethicone Copolyol" für Dimethicone, das heißt, für lineare, vollständig methylierte Siloxanpolymere, die endständig und/oder seitenständig mit mindestens einer CH₂CH₂O- und/oder mindestens einer CH₂CH(CH₃)O- und/oder mindestens einer CH(CH₃)CH₂O-Einheit substituiert sind.

Unter hydrophil modifizierten Silikonen werden erfindungsgemäß allgemeiner Polyorganosiloxane mit hydrophilen Substituenten verstanden, welche die Wasserlöslichkeit bzw. Grenzflächenaktivität der Silikone erhöhen. Die hydrophil modifizierten Silikone reduzieren die Klebrigkeit und hinterlassen ein frisches Hautgefühl. Unter den hydrophil modifizierten Silikonen sind solche bevorzugt, die sich mindestens in einer Menge von 0,5 Gew.-% bei 20°C in Wasser lösen. Entsprechende hydrophile Substituenten sind beispielsweise Hydroxy-, Hydroxyalkyl-, Polyalkylenglycol-Seitenketten, insbesondere Polyethylenglycol- oder Polyethylenglycol/Polypropylenglycol-Seitenketten, sowie ethoxylierte Ester-Seitenketten.

Vorzugsweise besitzt eine 0,2%ige wässrige Lösung der erfindungsgemäß verwendbaren hydrophil modifzierten Silikone bei 25°C eine Oberflächenspannung von kleiner gleich 40 mN/m, besonders bevorzugt von kleiner gleich 35 mN/m.

Erfindungsgemäß geeignet sind beispielsweise hydrophil modifizierte Silicon-Copolyole, insbesondere Dimethicone-Copolyole, die beispielsweise von Wacker-Chemie unter der Bezeichnung Belsil® DMC 6031 (PEG/PPG-25/25 Dimethicone), Belsil® DMC 6032, Belsil® DMC 6038 (Bis-PEG-15 Methyl Ether Dimethicone) oder Belsil^{®} DMC 3071 VP (Cetyl PEG/PPG-15/15 Butyl Ether Dimethicone), von Dow Corning unter der Bezeichnung DC 193, DC 5324 oder DC 5329 (jeweils PEG-12 Dimethicone), DC 2501 (BIS-PEG-18 Methyl Ether Dimethyl Silane) oder DC Q2-5220 (PEG/PPG-17/18 Dimethicone), von Degussa/Goldschmidt unter der Bezeichnung Abil EM 90 (Cetyl PEG/PPG-10/1 Dimethicone), Abil EM 97 (Bis-PEG/PPG-14/14 Dimethicone), Abil Care 85 (Bis-PEG/PPG-16/16 PEG/PPG 16/16 Dimethicone; Caprylic/Capric Triglyceride), Abil B 8832 (Bis-PEG/PPG-20/20-Dimethicone), Abil B 88183 oder Abil B 88184 (jeweils PEG/PPG-20/6-Dimethicone), von Noveon unter den Bezeichnungen Ultrasil DW-18 bzw. SilSense DW-18 Silicone (Dimethicone PEG-7 Isostearate), Ultrasil SW-12 bzw. SilSense DW-12 Dimethicone Copolyol Ester (Dimethicone PEG-7 Cocoate), Ultrasil DW-AV bzw. SilSense DW-AV Silicone (Dimethicone PEG-7 Avocadoate) oder Ultrasil DW-O bzw. SilSense DW-O Silicone (Dimethicone PEG-7 Olivate), von Shin-Etsu unter der Bezeichnung KF618 (PEG-6 Methyl Ether Dimethicone), KF6011 (PEG-11 Methyl Ether Dimethicone), KF6012 (PEG/PPG-20/22 Butyl Ether Dimethicone), KF6013 (PEG-9 Dimethicone), KF6015 (PEG-3 Dimethicone), KF6016 (PEG-9 Methyl Ether Dimethicone) oder KF6017 (PEG-10 Dimethicone), von Nippon Unicar Co. Ltd. unter der Bezeichnung FZ 2401 (PEG/PPG-20/22 Methyl Ether Dimethicone) und von Rhodia unter der Bezeichnung Mirasil DMCP93 (PEG/PPG-10/2 Dimethicone) im Handel erhältlich sind. Erfindungsgemäß kann auch ein beliebiges Gemisch der genannten Silicone eingesetzt werden.

Die erfindungsgemäß einsetzbaren hydrophil modifizierten Silikone besitzen in einer weiteren bevorzugten Ausführungsform einen HLB-Wert größer 7, besonders bevorzugt einen HLB-Wert von 8 bis 17 und insbesondere von 10 bis 15.

Die Menge des hydrophil modifizierten Silikons in den erfindungsgemäßen Zusammensetzungen beträgt, sofern vorhanden, vorzugsweise bis zu 5,0 Gew.-%, besonders bevorzugt von 0,1 bis 4,0 Gew.-%, insbesondere von 0,2 - 3,0 Gew.-%, bezogen auf das Gesamtgewicht der Zusammensetzung.

### Pflanzenextrakte

In einer erfindungsgemäßen Ausführungsform enthält die Zusammensetzung mindestens einen Pflanzenextrakt. Der Pflanzenextrakt kann beispielsweise durch Extraktion der gesamten Pflanze, aber auch ausschließlich durch Extraktion aus Blüten und/oder Blättern und/oder Samen und/oder anderen Pflanzenteilen, hergestellt werden. Erfindungsgemäß sind vor allem die Extrakte aus dem Meristem, also dem teilungsfähigen Bildungsgewebe der Pflanzen, und die Extrakte aus speziellen Pflanzen wie Grünem Tee, Hamamelis, Kamille, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Wiesenschaumkraut, Schafgarbe, Quendel, Thymian, Melisse, Hauhechel, Eibisch (Althaea), Veilchen, Blättern der Schwarzen Johannisbeere, Huflattich, Fünffingerkraut, Ginseng, Ingwerwurzel und Süßkartoffel als weiteres Pflanzenextrakt bevorzugt. Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesiumangereicherten Medium kultiviert wurden.

Als Pflanzenextrakt besonders bevorzugt sind die Extrakte aus Spirulina, Grünem Tee, Aloe Vera, Meristem, Hamamelis, Aprikose, Guave, Süßkartoffel, Limette, Mango, Kiwi, Gurke, Malve, Eibisch und Veilchen. Die erfindungsgemäßen Mittel können auch Mischungen aus mehreren, insbesondere aus zwei, verschiedenen Pflanzenextrakten enthalten. In einer besonders bevorzugten Ausführungsform ist in der erfindungsgemäßen Zusammensetzung einer der zuvor genannten Extrakte, insbesondere ein Extrakt aus Aloe vera, in einer Menge von 0,005 bis 0,1 Gew.-% enthalten.

In einer weiteren bevorzugten Ausführungsform enthalten die erfindungsgemäßen Zusammensetzungen einen Extrakt aus den Bohnen von Kakao (Theobroma cacao) und/oder einen Extrakt aus den Blättern der Pfefferminze (Mentha piperita).

Als Extraktionsmittel zur Herstellung der Pflanzenextrakte können beispielsweise Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol, Propylenglykol und Butylenglykol und zwar sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen. Die Wasserdampfdestillation fällt erfindungsgemäß unter die bevorzugten Extraktionsverfahren. Die Extraktion kann aber gegebenenfalls auch in Form von Trockenextraktion erfolgen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch. Je nach Wahl der Extraktionsmittel kann es bevorzugt sein, den Pflanzenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-//caprinsäureglyceride, Polyoxyethylenglycerinmonolaurat und Polyoxyethylenglykolkokosfettsäureglyceride.

Weiterhin kann es bevorzugt sein, in den erfindungsgemäßen Mitteln Mischungen aus mehreren, insbesondere aus zwei, verschiedenen Pflanzenextrakten einzusetzen.

Hinsichtlich der erfindungsgemäß verwendbaren Pflanzenextrakte wird weiterhin auf die Extrakte hingewiesen, die in der auf Seite 44 der 3. Auflage des Leitfadens zur lnhaltsstoffdeklaration kosmetischer Mittel, herausgegeben vom Industrieverband Körperpflege- und Waschmittel e.V. (IKW), Frankfurt, beginnenden Tabelle aufgeführt sind.

### Wasserlösliche einwertige Alkohole

Die erfindungsgemäßen Zusammensetzungen sind erfindungsgemäß vorzugsweise völlig frei von wasserlöslichen einwertigen Alkoholen, da diese zur Hautreizung führen können. Falls wasserlösliche einwertige Alkohole enthalten sind, so sind diese erfindungsgemäß vorzugsweise nur in sehr geringen Mengen, vorzugsweise in einer Menge von weniger als 0,5 Gew.-%, besonders bevorzugt in einer Menge von weniger als 0,3 oder 0,2 Gew.-%, vor allem in einer Menge von weniger als 0,1 Gew.-%, enthalten. Unter Wasserlöslichkeit versteht man erfindungsgemäß, dass sich wenigstens 5 Gew.-% des Alkohols bei 20 °C klar lösen. Beispiele für wasserlösliche einwertige Alkohole sind Ethanol, Propanol, Isopropanol oder Benzylalkohol.

Ebenso wie wasserlösliche einwertige Alkohole sind vorzugsweise auch andere organische Lösungsmittel, insbesondere solche mit einem Molekulargewicht von weniger als 200 D, in den erfindungsgemäßen Zusammensetzungen nur in sehr geringen Mengen, vorzugsweise in einer Menge von weniger als 0,5 Gew.-%, besonders bevorzugt in einer Menge von weniger als 0,3 oder 0,2 Gew.-%, vor allem in einer Menge von weniger als 0,1 Gew.-% oder gar nicht enthalten.

### Aminosäuren, Peptide, Proteine und deren Derivate

Weiterhin können die erfindungsgemäßen Zusammensetzungen Monomere, Oligomere und Polymere von Aminosäuren und N-C₂-C₂₄-Acylaminosäuren sowie Ester und/oder physiologisch verträgliche Metallsalze dieser Substanzen enthalten.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Omithin, Phenylalanin, Prolin, Pyroglutaminsäure, Sarcosin, Serin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, , Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Die Oligomere der Aminosäuren und/oder der N-C₂-C₂₄-Acylaminosäuren sind ausgewählt aus Di-, Tri-, Tetra-, Penta-, Hexa- oder Pentadecapeptiden, die acyliert und/oder verestert sein können. Erfindungsgemäß bevorzugte, gegebenenfalls acylierte und/oder veresterte Dipeptide sind Tyr-Arg (Dipeptide-1), Val-Trp (Dipeptide-2), Asn-Phe, Asp-Phe, N-Palmitoyl-ß-Ala-His, N-Acetyl-Tyr-Arghexyldecylester (z. B. Calmosensine von Sederma), Carnosin (ß-Ala-His) und N-Palmitoyl-Pro-Arg. Erfindungsgemäß bevorzugte, gegebenenfalls acylierte und/oder veresterte Tripeptide sind Lys-Pro-Val, Gly-His-Lys (Tripeptide-1, z. B. Omega-CH-Aktivator von GfN), N-Palmitoyl-Gly-His-Lys, Gly-Lys-His, Tyr-Tyr-Val, Tyr-Val-Tyr, Val-Tyr-Val (Tripeptide-2), Gly-His-Arg (Tripeptide-3), N-Myristoyl-Gly-His-Arg (z. B. Collasyn 314-GR von Therapeutic Peptide Inc.), Tripeptide-4 (z. B. ATPeptide, zu beziehen über IMPAG), His-Ala-Orn, Lys-Phe-Lys, N-Elaidoyl-Lys-Phe-Lys und N-Acetyl-Arg-Lys-Arg-NH₂. Erfindungsgemäß bevorzugte, gegebenenfalls acylierte und/oder veresterte Tetrapeptide sind Val-Val-Arg-Pro, Gly-Gln-Pro-Arg, Gly-Gln-Arg-Pro und N-Palmitoyl-Gly-Gln-Pro-Arg. Erfindungsgemäß bevorzugte, gegebenenfalls acylierte und/oder veresterte Pentapeptide sind Lys-Thr-Thr-Lys-Ser, N-Palmitoyl-Lys-Thr-Thr-Lys-Ser, N-Palmitoyl-Tyr-Gly-Gly-Phe-Met, Val-Val-Arg-Pro-Pro und N-Palmitoyl-Tyr-Gly-Gly-Phe-Leu. Erfindungsgemäß bevorzugte, gegebenenfalls acylierte und/oder veresterte Hexapeptide sind Palmitoyl-Val-Gly-Val-Ala-Pro-Gly (Biopeptide EL von Sederma), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (Z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1).

Es kann erfindungsgemäß besonders bevorzugt sein, ein Gemisch aus mindestens zwei Oligopeptiden einzusetzen. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z. B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich und ist erfindungsgemäß ebenfalls besonders bevorzugt.

Die Polymere der Aminosäuren und/oder der N-C₂-C₂₄-Acylaminosäuren sind ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®*} (Cognis), Lexein^{®} (Inolex) und Crotein^{®} (Croda). Besonders bevorzugt sind Sojaproteinhydrolysate, z. B. die Handelsprodukte Phytokine von Coletica oder Ridulisse C von Silab. Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Erfindungsgemäß einsetzbar sind auch kationisierte Proteinhydrolysate. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.
Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen. Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom *denV*-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.
In den erfindungsgemäßen Zusammensetzungen sind die Photosome™ oder Ultrasome™ in Mengen von 0,1-10 Gew.-%, bevorzugt 0,5-5,0 Gew.-% und besonders bevorzugt 1,0-4,0 Gew.-%, bezogen auf das gesamte Mittel, enthalten.
In den erfindungsgemäßen Zusammensetzungen sind die Monomere, Oligomere oder Polymere von Aminosäuren, N-C₂-C₂₄-Acylaminosäuren und/oder den Estern und/oder den physiologisch verträglichen Metallsalzen dieser Substanzen in Mengen von 0,01 - 10 Gew.-%, bevorzugt 0,1 - 5 Gew.-% und besonders bevorzugt 0,1 - 3 Gew.-%, jeweils bezogen auf die gesamte Zusammensetzung, enthalten.

In einer weiteren bevorzugten Ausführungsform liegen die Monomeren, Oligomeren und Polymeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, Talkum, Microsponges, modifizierten Stärken und Stärkederivaten, kristalliner Cellulose, Cellulosepulvem, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

### Oligonucleotide

Die erfindungsgemäßen Zusammensetzungen können weiterhin Oligonukleotide enthalten. Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).
Ein erfindungsgemäß besonders bevorzugtes Oligonucleotid ist das Thymidin-Dinucleotid.
In den erfindungsgemäßen Zusammensetzungen sind die DNA-Oligonucleotide oder RNA-Oligonucleotide in Mengen von 0,0001 -5 Gew.-%, bevorzugt 0,001 -1,0 Gew.% und besonders bevorzugt 0,01 - 0,5 Gew.-%, bezogen auf das gesamte Mittel, enthalten.

### Betain-Verbindungen

Die erfindungsgemäßen Zusammensetzungen können weiterhin Betain-Verbindungen enthalten. Erfindungsgemäß eingesetzte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl.
Die Betainverbindungen sind in den erfindungsgemäßen Mitteln in einer Gesamtmenge von 0,05 bis 5 Gew.-%, bevorzugt 0,1 bis 3 Gew.%, besonders bevorzugt 0,5 bis 2 Gew.-%, jeweils bezogen auf die gesamte Zusammensetzung, enthalten.

### Zuckerbestandteile

Weiterhin können die erfindungsgemäßen Zusammensetzungen neben den hydrophob modifizierten Polysacchariden weitere Zuckerbestandteile ausgewählt aus Mono-, Oligo- und/oder Polysacchariden und/oder deren Derivaten enthalten.

Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin. Bevorzugt sind Glucose, Fructose, Galactose, Arabinose und Fucose; Glucose ist besonders bevorzugt.

Erfindungsgemäß geeignete Oligosaccharide sind aus zwei bis zehn Monosaccharideinheiten zusammengesetzt, z. B. Saccharose, Lactose oder Trehalose. Ein besonders bevorzugtes Oligosaccharid ist Saccharose. Ebenfalls besonders bevorzugt ist die Verwendung von Honig, der überwiegend Glucose und Saccharose enthält.

Erfindungsgemäß geeignete Polysaccharide sind aus mehr als zehn Monosaccharideinheiten zusammengesetzt. Bevorzugte Polysaccharide sind die aus α-D-Glucose-Einheiten aufgebauten Stärken sowie Stärkeabbauprodukte wie Amylose, Amylopektin und Dextrine. Erfindungsgemäß besonders vorteilhaft sind chemisch und/oder thermisch modifizierte Stärken, z. B. Hydroxypropylstärkephosphat, Dihydroxypropyldistärkephosphat oder die Handelsprodukte Dry Flo^{®}. Weiterhin bevorzugt sind Dextrane sowie ihre Derivate, z. B. Dextransulfat. Ebenfalls bevorzugt sind nichtionische Cellulose-Derivate, wie Methylcellulose oder Hydroxyethylcellulose, sowie kationische Cellulose-Derivate, z. B. die Handelsprodukte Celquat^{®} und Polymer JR^{®}, und bevorzugt Celquat^{®} H 100, Celquat^{®} L 200 und Polymer JR^{®} 400 (Polyquaternium-10) sowie Polyquatemium-24. Weitere bevorzugte Beispiele sind Polysaccharide aus Fucose-Einheiten, z. B. das Handelsprodukt Fucogel^{®}. Besonders bevorzugt sind die aus Aminozuckereinheiten aufgebauten Polysaccharide, insbesondere Chitine und ihre deacetylierten Derivate, die Chitosane, und Mucopolysaccharide. Zu den erfindungsgemäß bevorzugten Mucopolysacchariden gehören Hyaluronsäure und ihre Derivate, z. B. Natriumhyaluronat oder Dimethylsilanolhyaluronat, sowie Chondroitin und seine Derivate, z. B. Chondroitinsulfat. Als Verdicker einsetzbar sind neben den zuvor genannten Celluloseethern etwa auch Gelatine, Pflanzengumme wie Agar-Agar, Guar-Gum, Alginate, Xanthan-Gum, Gummi arabicum, Karaya-Gummi oder Johannisbrotkernmehl.

Die zusätzlichen Zuckerbestandteile sind, falls vorhanden, vorzugsweise in Mengen bis zu 5 Gew.-% in den erfindungsgemäßen Zusammensetzungen enthalten.

### Weitere Polymere

Außer den hydrophob modifizierten Polysacchariden und den zuvor genannten weiteren hydrophob modifizierten Polymeren können weitere Polymere, insbesondere ausgewählt aus kationischen, anionischen und nichtionischen Polymeren, enthalten sein.

Unter den kationischen Polymeren bevorzugt sind Polysiloxane mit quaternären Gruppen, z. B. die Handelsprodukte Q2-7224 (Dow Coming), Dow Corning^{®} 929 Emulsion (mit Amodimethicone), SM-2059 (General Electric), SLM-55067 (Wacker) sowie Abil^{®}-Quat 3270 und 3272 (Th. Goldschmidt).

Bevorzugte anionische Polymere enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Ganz besonders bevorzugte anionische Polymere enthalten als alleiniges Monomer oder als Comonomer 2-Acrylamido-2-methylpropansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppen ganz oder teilweise als Natrium-, Kalium-, Ammonium-, Mono- oder Triethanolammonium-Salz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Weitere bevorzugte anionische Homo- und Copolymere sind unvernetzte und vernetzte Polyacrylsäuren. Dabei können Allylether von Pentaerythrit, von Sucrose und von Propylen bevorzugte Vernetzungsagentien sein.

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Die genannten weiteren Polymere sind, falls vorhanden, vorzugsweise in Mengen bis zu 2 Gew.-% enthalten.

### Hydroxy- und Ketocarbonsäuren

In einer weiteren bevorzugten Ausführungsform enthalten die erfindungsgemäßen Zusammensetzungen mindestens eine α-Hydroxycarbonsäure, α-Ketocarbonsäure oder β-Hydroxycarbonsäure oder deren Ester-, Lacton- oder Salzform. Erfindungsgemäß geeignete α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbemsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate sind in Mengen von 0,1 - 10 Gew.-%, bevorzugt 0,5 - 5 Gew.-%, jeweils bezogen auf die gesamte Zusammensetzung, enthalten.

### Verdickungsmittel

Flüssige und gelförmige Darreichungsformen der erfindungsgemäßen Zusammensetzungen können neben oder anstelle der zuvor genannten als Verdickungsmittel geeigneten Polysaccharide etwa auch verdickende Polymere auf Basis von Polyacrylaten enthalten, die gewünschtenfalls vernetzt sein können, oder auf Basis von Polyacrylamiden oder sulfonsäuregruppenhaltigen Polyacrylaten, z. B Sepigel^{®} 305 oder Simulgel^{®} EG. Weiterhin können auch anorganische Verdicker, z. B. natürliche und synthetische Tone und Schichtsilikate, z. B. Bentonit, Hectorit, Montmorillonit oder Laponite^{®}, sowie vollsynthetische Hydrokolloide wie z. B. Polyvinylalkohol, und außerdem Ca-, Mg- oder Zn-Seifen von Fettsäuren in erfindungsgemäßen Zusammensetzungen enthalten sein.

### Hydrotrope

Erfindungsgemäß können ferner bestimmte Hydrotrope, wie beispielsweise Polyole, in geringen Mengen eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind Glycerin, Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin; Dialkoholamine, wie Diethanolamin oder 2-Amino-1,3-propandiol. In einer erfindungsgemäß bevorzugten Ausführungsform wird Glycerin in einer Menge von 0,5 bis 5 Gew.-% eingesetzt.

### Konservierungsmittel

Als Konservierungsmittel eignen sich beispielsweise Phenoxyethanol, Formaldehydlösung, Parabene, insbesondere Methyl- oder Propylparaben, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton.

### Vitamine

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Zubereitungen enthalten die Vitamin A-Komponente bevorzugt in Mengen von 0,05 - 1 Gew.-%, bezogen auf die gesamte Zusammensetzung.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf das gesamte Mittel, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist. 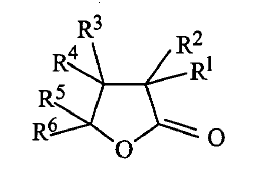
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, - dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05-1 Gew.-%, bezogen auf die gesamte Zusammensetzung, enthalten.
Unter Vitamin F werden üblicherweise essentielle Fettsäuren, insbesondere Linolsäure, Linolensäure und Arachidonsäure, verstanden.
Vitamin H ist eine andere Bezeichnung für Biotin oder Vitamin B₇ (siehe oben).

Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.
Vitamin A-palmitat (Retinylpalmitat), Panthenol, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

### Flavonoide

In einer weiteren bevorzugten Ausführungsform enthalten die erfindungsgemäßen Zusammensetzungen mindestens ein Flavonoid oder mindestens einen Flavonoid-reichen Pflanzenextrakt.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

### Isoflavonoide

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.

Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin. Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder lsoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isofiavonoidreicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß werden die Isoflavonoide in Mengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, eingesetzt.

### Polyphenole

In einer weiteren bevorzugten Ausführungsform enthalten die erfindungsgemäßen Zusammensetzungen mindestens ein Polyphenol oder einen Polyphenol-reichen Pflanzenextrakt. Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Fam-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay, ein Extrakt aus den Kernen der Chardonnay-Traube, erhältlich von der Firma Croda. Erfindungsgemäß werden die Polyphenole in Mengen von 0,001 bis 10 Gew.-%, bevorzugt 0,005 bis 5 Gew.-% und besonders bevorzugt 0,01 bis 3 Gew.-%, jeweils bezogen auf die gesamte kosmetische Zusammensetzung, eingesetzt.

### Ubichinol-Verbindungen

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (II) auf: 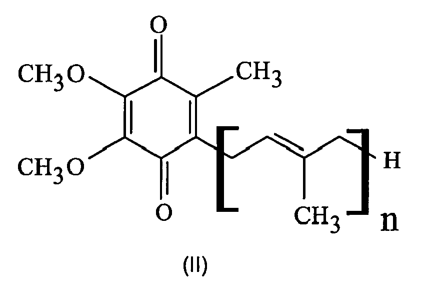 mit n = 6, 7, 8, 9 oder 10.
Besonders bevorzugt ist das Ubichinon der Formel (II) mit n = 10, auch bekannt als Coenzym Q10.

Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

### UV-Filtersubstanzen

In einer weiteren bevorzugten Ausführungsform enthalten die erfindungsgemäßen Zusammensetzungen mindestens eine anorganische und/oder mindestens eine organische UV-Filtersubstanz.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.

Die erfindungsgemäß verwendeten organischen UV-Filter sind ausgewählt aus den Derivaten von Dibenzoylmethan, Zimtsäureestern, Diphenylacrylsäureestern, Benzophenon, Campher, p-Aminobenzoesäureestem, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethylhexyloxy)-2-hydroxy]-phenyl}-6-[4-(2-methoxyethylcarboxyl)-phenylamino]-1,3,5-triazin, 2,4-Bis-{[4-(3-(2- propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis- {[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtem herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

### Sebumregulierende Wirkstoffe

In einer weiteren bevorzugten Ausführungsform enthalten die erfindungsgemäßen Zusammensetzungen mindestens einen sebumregulierenden Wirkstoff. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCI: Aqua, Hydrolyzed Yeast Protein, Pyridoxine, Niacinamide, Glycerin, Panthenol, Biotin) und Antifettfaktor^{®} COS-218/2-A (Cosmetochem, INCI: Aqua, Cetyl-PCA, PEG-8 Isolauryl Thioether, PCA, Cetyl Alcohol).
Die sebumregulierenden Wirkstoffe sind in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

### Deodorantien

Die erfindungsgemäßen Zusammensetzungen enthalten in einer besonderen erfindungsgemäßen Ausführungsform mindestens einen Deodorant-Wirkstoff. Erfindungsgemäß als Deodorant-Wirkstoffe geeignet sind Duftstoffe, antimikrobielle, antibakterielle oder keimhemmende Stoffe, enzymhemmende Stoffe, Antioxidantien und Geruchsadsorbentien.

Geeignet sind insbesondere Organohalogenverbindungen sowie -halogenide, quartäre Ammoniumverbindungen und Zinkverbindungen. Bevorzugt sind Chlorhexidin und Chlorhexidingluconat, Benzalkoniumhalogenide und Cetylpyridiniumchlorid. Desweiteren sind Natriumbicarbonat, Natriumphenolsulfonat und Zinkphenolsulfonat, die Bestandteile des Lindenblütenöls, Phenoxyethanol, Triclosan (Irgasan^{®} DP300) oder Triethylcitrat einsetzbar.

Als Enzym-hemmende Stoffe bevorzugt sind Inhibitoren für Enzyme der axillaren Keimflora, die bei der Entstehung von Körpergeruch involviert sind. Hierbei handelt es sich vorzugsweise um Inhibitoren von Lipasen, Arylsulfatasen (s. WO 01/99376), β-Glucoronidasen (s. WO 03/039505), 5-α-Reduktasen und Aminoacylasen.

Weitere antibakteriell wirksame Deodorant-Wirkstoffe sind Parfümöle, Ethylhexylglycerinether (Sensiva^{®} SC 50), Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide.

Weiterhin geeignet als Deodorant-Wirkstoff sind wasserlösliche Polyole, ausgewählt aus wasserlöslichen Diolen, Triolen und höherwertigen Alkoholen sowie Polyethylenglycolen. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Pentandiole, z. B. 1,2-Pentandiol, sowie Hexandiole, z. B. 1,6-Hexandiol. Weiterhin bevorzugt geeignet sind Glycerin und technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, weiterhin 1,2,6-Hexantriol sowie Polyethylenglycole (PEG) mit einem durchschnittlichen Molekulargewicht von 100 bis 1.000 Dalton, beispielsweise PEG-400, PEG-600 oder PEG-1000. Weitere geeignete höherwertige Alkohole sind die C₄-, C₅- und C₆-Monosaccharide und die entsprechenden Zuckeralkohole, z. B. Mannit oder Sorbit.

### Antioxidantien

Die erfindungsgemäßen Zusammensetzungen können ferner Antioxidantien enthalten, zum Beispiel Imidazole (z. B. Urocaninsäure) und deren Derivate, Peptide wie D,L-Carnosin, D-Carnosin, L-Camosin und deren Derivate (z. B. Anserin), Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, das Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Katalase, Superoxid-Dismutase, Zink und dessen Derivate (z. B. ZnO, ZnSO₄), Selen und dessen Derivate (z. B. Selen-Methionin), Stilbene und deren Derivate (z. B. Stilbenoxid, trans-Stilbenoxid) und die als Antioxidans geeigneten Derivate (Salze, Ester, Ether, Zucker, Nukleotide, Nukleoside, Peptide und Lipide) dieser Wirkstoffe.

### Weitere Wirk-, Hilfs- und Zusatzstoffe

Die erfindungsgemäßen Mittel können weitere Wirk-, Hilfs- und Zusatzstoffe enthalten, beispielsweise Taurin; Caomint (Solabia); Allantoin; Bisabolol; Ceramide und Pseudoceramide; Triterpene, insbesondere Triterpensäuren wie Ursolsäure, Rosmarinsäure, Betulinsäure, Boswelliasäure und Bryonolsäure; monomere Catechine, besonders Catechin und Epicatechin, Leukoanthocyanidine, Catechinpolymere (Catechin-Gerbstoffe) sowie Gallotannine; Pflanzenglycoside; Strukturanten wie Maleinsäure und Milchsäure; Dimethylisosorbid; Lösungsmittel, Quell- und Penetrationsstoffe wie Ethanol, Isopropanol, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate; Parfümöle, Pigmente sowie Farbstoffe zum Anfärben des Mittels; Substanzen zur Einstellung des pH-Wertes, z. B. α- und β-Hydroxycarbonsäuren; Komplexbildner wie EDTA, NTA, β-Alanindiessigsäure und Phosphonsäuren; Trübungsmittel wie Latex, Styrol/PVP- und Styrol/Acrylamid-Copolymere; Perlglanzmittel wie Ethylenglykolmono- und -distearat sowie PEG-3-distearat; Treibmittel wie Propan-Butan-Gemische, N₂O, Dimethylether, CO₂ und Luft; MMP-1-inhibierende Substanzen, insbesondere ausgewählt aus Photolyase und/oder T4 Endonuclease V, Propylgallat, Precocenen, 6-Hydroxy-7-methoxy-2,2-dimethyl-1(2H)-benzopyran und 3,4-Dihydro-6-hydroxy-7-methoxy-2,2-dimethyl-1(2H)-benzopyran; entzündungshemmende Substanzen; Feststoffe, ausgewählt aus Kieselsäuren, z. B. Aerosil^{®}-Typen, Kieselgelen, Siliciumdioxid, Tonen, z. B. Bentonite oder Kaolin, Magnesiumaluminiumsilikaten, z. B. Talkum, Bornitrid, Titandioxid, das gewünschtenfalls beschichtet sein kann, gegebenenfalls modifizierten Stärken und Stärkederivaten, Cellulosepulvern und Polymerpulvern; Anti-Akne-Wirkstoffe sowie Keratolytika.

In einer erfindungsgemäß besonders bevorzugten Ausführungsform wird eine Mischung aus Ethylcellulose in einer Menge von 0,1 - 5,0, bevorzugt 0,5 - 2,0 Gew.-%, Wachsen, die im Bereich von 35 - 95°C schmelzen, in einer Menge von 0,1 - 5,0, bevorzugt 1,0 - 4,0 Gew.-% und polaren Ölen, vorzugsweise verzweigten Alkanolen umfassend einen linearen C₈₋₁₈-Alkanol, der durch C₄₋₁₂-Alkylreste ein- oder mehrfach, vorzugsweise einfach, insbesondere am Kohlenstoffatom 2 substituiert ist, wie beispielsweise 2-Hexyldecanol, 2-Octyldodecanol, Isotridecanol oder Isohexadecanol, in einer Menge von 1 - 40, bevorzugt 2 - 35, besonders bevorzugt 5 - 30 Gew.-% eingesetzt.

### Ausführungsbeispiele

### Beispiel 1: Herstellverfahren

Zunächst wird der Wachs in der Ethylcellulose aufgelöst (70°C, 5 Minuten). Anschließend wird die so erhaltene Lösung (Quellung - Oleogel) mit dem Öl (beispielsweise 2-Octyldodecanol) vermischt durch 30 Minuten Rühren bei 80°C (Dissolverscheibe, 1000 U/min). Diese Lösung wird für einen Tag bei Raumtemperatur ruhen gelassen. Anschließend wird die Wasserphase bereitgestellt, indem Salz und/oder Polymer bei Raumtemperatur hinzugegeben werden. Jetzt werden Öl- und Wasserphase jeweils auf 70°C erhitzt und danach die Wasserphase portionsweise unter Rühren zur Ölphase hinzugegeben (Dissolverscheibe, 1000 U/min). Anschließend erfolgt Abkühlung auf Raumtemperatur unter Rühren.

### Beispiel 2: Rezepturen

| Substanz | Chem. Name | 1 | 2 | 3 | 4 | 5 |
|---|---|---|---|---|---|---|
| Hercules EC-N7 | Ethylcellulose | 1,5 | 1,5 | 1,5 | 1,5 | 1,5 |
| Eutanol G | 2-Octyldodecanol | 28,5 | 28,5 | 28,5 | 28,5 | 28,5 |
| Cutina CP | Cetylpalmitat | 3 | | | | |
| Lanolin | Wollwachs | | 3 | | | |
| Ozokerit | | | | 3 | | |
| Bienenwachs | | | | | 3 | |
| Paraffinwachs | SMP:43-46°C | | | | | 3 |
| Wasser (VE) | | ad 100 | ad 100 | ad 100 | ad 100 | ad 100 |

### Beispiel 3: Rezepturen

| Produkt | Menge in Gew.-% | | | | | | | | | |
|---|---|---|---|---|---|---|---|---|---|---|
| Rezeptur | 6 | 7 | 8 | 9 | 10 | 11 | 12 | 13 | 14 | 15 |
| Ethylcellulose (Hercules EC-N7) | 1,5 | 1,5 | 1,0 | 0,8 | | | 0,5 | 1,5 | | 1,5 |
| Ethylcellulose (Hercules EC-N10) | | | | | 1,5 | 1,2 | 0,7 | | 1,0 | |
| Ozokerite | 3 | 3 | 3 | 3 | | | | 2 | 2 | 2 |
| Beeswax | | | | | 3 | 3 | 3 | 1 | 1 | 1 |
| 2-Octyldodecanol | 13,5 | 13,5 | 13,5 | 28,5 | 28,5 | 13,5 | 13,5 | 28,5 | 28,5 | 28,5 |
| Isopropyl Palmitate | 15 | | | | | 15 | | | | |
| Ethylhexyl Stearate | | 15 | 15 | | | | 15 | | | |
| Tocopheryl Acetate | 1 | 1 | 1 | 1 | 0,5 | 0,5 | 0,5 | | | |
| Glycerin | | | | 2,0 | 2,0 | 2,0 | 2,0 | 2,0 | 5,0 | 5,0 |
| Preservative | q.s. | q.s. | q.s. | q.s. | q.s. | q.s. | q.s. | q.s. | q.s. | q.s. |
| Parfume | q.s. | q.s. | q.s. | q.s. | q.s. | q.s. | q.s. | q.s. | q.s. | q.s. |
| Water (VE) | ad 100 | ad 100 | ad 100 | ad 100 | ad 100 | ad 100 | ad 100 | ad 100 | ad 100 | ad 100 |

| Produkt | Rohstoff | Hersteller |
|---|---|---|
| Hercules EC-N7 | Ethylcellulose | Hercules |
| Hercules EC-N10 | Ethylcellulose | Hercules |
| Eutanol G | 2-Octyldodecanol | Cognis |
| Cutina CP | Cetylpalmitat | Cognis |
| Super Hartolan | Lanolin Alcohol | Croda |
| Ozokerite Wax | Ozokerite | Kahl |
| Beeswax | Beeswax | Kahl |
| IPP | Isopropyl Palmitate | Cognis |
| Cetiol 868 | Ethylhexyl Stearate | Cognis |

## Patentansprüche

1. W/O-Emulsion, enthaltend folgende Bestandteile:
a) mindestens eine C₁₋₄-Alkylcellulose,
b) mindestens einen Wachs,
c) mindestens ein Öl,
d) Wasser,
**dadurch gekennzeichnet, dass** die Emulsion Tenside und Emulgatoren mit einem Molekulargewicht kleiner als 1000 g/mol in einer Menge von weniger als 0,2 Gew.-% sowie wasserlösliche einwertige Alkohole in einer Menge von weniger als 0,5 Gew.-% enthält.

2. W/O-Emulsion nach Anspruch 1, **dadurch gekennzeichnet, dass** die C₁₋₄-Alkylcellulose in einer Menge von 0,1 - 5,0 Gew.-% enthalten ist.

3. W/O-Emulsion nach Anspruch 1 oder 2, **dadurch gekennzeichnet, dass** der Wachs in einer Menge von 0,1 bis 5,0 Gew.-% enthalten ist.

4. W/O-Emulsion nach einem der Ansprüche 1 bis 3, **dadurch gekennzeichnet, dass** das Öl in einer Menge von 1 bis 30 Gew.-% enthalten ist.

5. W/O-Emulsion nach einem der Ansprüche 1 bis 4, **dadurch gekennzeichnet, dass** es sich bei der C₁₋₄-Alkylcellulose um Ethylcellulose handelt.

6. W/O-Emulsion nach einem der Ansprüche 1 bis 5, **dadurch gekennzeichnet, dass** es sich bei dem Wachs um einen pflanzlichen Wachs, einen tierischen Wachs, einen Mineralwachs, einen chemisch modifizierten Wachs oder einen synthetischen Wachs handelt.

7. W/O-Emulsion nach Anspruch 6, **dadurch gekennzeichnet, dass** es sich bei dem Wachs um Ozokerit, Cetylpalmitat, Wollwachs, Bienenwachs oder Paraffinwachs handelt.

8. W/O-Emulsion nach einem der Ansprüche 1 bis 7, **dadurch gekennzeichnet, dass** es sich bei dem Öl um ein polares oder unpolares Öl handelt.

9. W/O-Emulsion nach Anspruch 8, **dadurch gekennzeichnet, dass** es sich bei dem polaren Öl um einen verzweigten Alkanol umfassend einen linearen C₈₋₁₈-Alkanol, der durch C₄₋₁₂-Alkylreste ein- oder mehrfach substituiert ist, handelt.

10. W/O-Emulsion nach einem der Ansprüche 1 bis 9, **dadurch gekennzeichnet, dass** sie mindestens ein wasserlösliches Polymer und/oder mindestens ein Salz enthält.

11. W/O-Emulsion nach Anspruch 10, **dadurch gekennzeichnet, dass** das wasserlösliche Polymer Polyethylenglykol und/oder Polyvinylpyrrolidon mit einem mittleren Molekulargewicht im Bereich von 5000 bis 50000 D umfasst.

12. W/O-Emulsion nach Anspruch 10 oder 11, **dadurch gekennzeichnet, dass** das Salz ein anorganisches Salz umfasst.

13. W/O-Emulsion nach einem der Ansprüche 1 bis 12, **dadurch gekennzeichnet, dass** sie Liposomen, uni-, pauci- und/oder multilamellare Vesikel enthält.

14. Verwendung einer W/O-Emulsion nach einem der Ansprüche 1 bis 13 zur Behandlung der Gesichtshaut.

15. Verwendung einer W/O-Emulsion nach einem der Ansprüche 1 bis 13 zur Behandlung von empfindlicher oder trockener Haut.

16. Verwendung einer W/O-Emulsion nach einem der Ansprüche 1 bis 13 zur Behandlung von Haut in empfindlichen oder sensitiven Bereichen, insbesondere im Bereich der Augen.

17. Verwendung einer W/O-Emulsion nach einem der Ansprüche 1 bis 13 zum Transport von Wirkstoffen in die Haut.

18. Verwendung einer W/O-Emulsion nach einem der Ansprüche 1 bis 13 zur Behandlung extrinsisch und/oder intrinsisch gealterter Haut.

19. Kosmetische oder pharmazeutische Zusammensetzung enthaltend eine W/O-Emulsion nach einem der Ansprüche 1 bis 13.

20. Kosmetische oder pharmazeutische Zusammensetzung nach Anspruch 19, **dadurch gekennzeichnet, dass** es sich um eine Augencreme oder Augensalbe handelt.
